# EUROPÄISCHE PATENTSCHRIFT

(11) **EP 3 141 204 B1**
(45) Veröffentlichungstag und Bekanntmachung des Hinweises auf die Patenterteilung: **28.07.2021**
(21) Anmeldenummer: 15184689.6
(22) Anmeldetag: 10.09.2015
(51) Int. Cl.: A61B 18/04

(54) **ABLATIONSSYSTEM ZUR GROSSFLÄCHIGEN OBERFLÄCHENKOAGULATION BIOLOGISCHER GEWEBE**
ABLATION SYSTEM FOR THE LARGE-SCALE SURFACE COAGULATION OF BIOLOGICAL TISSUES
SYSTEME D'ABLATION DESTINE A LA COAGULATION EXTENSIVE DE TISSUS BIOLOGIQUES

(43) Veröffentlichungstag der Anmeldung: 15.03.2017
(73) Patentinhaber: Erbe Elektromedizin GmbH, 72072 Tübingen (DE)
(72) Erfinder: Enderle, Markus, 72070 Tübingen (DE); Fischer, Klaus, 72202 Nagold (DE); Stäbler, Thomas, 72070 Tübingen (DE)
(74) Vertreter: Rüger Abel Patentanwälte PartGmbB

(56) Entgegenhaltungen:
- EP-A1- 1 090 597
- WO-A1-2008/090004
- US-A1- 2001 049 509
- US-A1- 2005 015 086
- US-A1- 2005 118 350
- US-A1- 2010 125 164
- US-A1- 2011 184 408

## Beschreibung

Die Erfindung betrifft ein Ablationssystem, insbesondere zur großflächigen Mucosaablation.

Zur therapeutischen Behandlung insbesondere der Magenschleimhaut, beispielsweise zur Tumorresektion oder auch zur Änderung des Ess- und Gewichtsverhaltens des Patienten, kann es erforderlich sein, scharf umrissene Bereiche der Mucosa und der Submucosa großflächig abzutragen und/oder zu koagulieren, wobei die Koagulationstiefe in dem behandelten Bereich möglichst einheitlich sein soll, so dass die Muscularis nicht zerstört wird. Die therapeutische Mucosaablation wird typischerweise mittels Endoskop durchgeführt, wobei zur Ablation spezielle Sonden zur Anwendung kommen können.

Beispielsweise ist aus der WO 2011/022069 A ein Endoskop mit einer Endkappe bekannt, die auf die Mucosa aufzusetzen ist und innerhalb derer eine Argon-Plasma-Koagulation durchgeführt wird. Die Kappe soll den Einwirkungsbereich der Argon-Plasma-Koagulation begrenzen.

Die US 8 641 711 B2 beschreibt ein Instrument zur Abtragung von Gewebeschichten von Hohlorganen, wobei das Instrument einen elektrisch aktiven Kopf mit Elektroden aufweist. An dem Kopf ist ein expandierbares Element vorgesehen, um den Kopf in Bezug auf die gegenüberliegende Gewebewand definiert zu positionieren.

Die US 2004/0215180 A1 offenbart eine Ablationssonde mit mehreren, von dem distalen Ende der Sonde abstehenden drahtartigen Einzelelektroden, die gemeinsam an einen elektrischen Leiter angeschlossen sind und über diesen von einem Stromgenerator mit einem die Ablation bewirkenden Strom versorgt werden.

Die US 2003/0181900 A1 veranschaulicht eine Sonde zur Kontaktkoagulation mit mehreren Elektroden, die in Betrieb an dem Gewebe anliegend zeitlich nacheinander nach einem bestimmten Schaltschema mit einem HF-Generator verbunden werden. Außerdem offenbart die US 2005/015086 A1 eine Sonde mit mehreren Elektroden, die über einen Auswahlschalter in verschiedenen Kombinationen mit einer elektrischen Quelle verbunden werden können. Dadurch können ein oder mehrere Plasmastrahlen erzeugt werden.

Weiterer relevanter Stand der Technik wird in den Dokumenten US 2005/015086 A1, US 2005/118350 A1, US 2011/184408 A1 und WO 2008/090004 A1 offenbart.

Zur simultanen chirurgischen Behandlung von biologischem Gewebe mittels mehrerer Plasmafunkensonden beschreibt die DE 10 2005 007 769 A1 ein System mit mehreren monopolaren Instrumenten, die über Hochspannungsschalter an einen einzigen HF-Generator angeschlossen sind, wobei die Schalter abwechselnd geschlossen werden, so dass die einzelnen Instrumente intermittierend HF-Strom erhalten.

Es ist Aufgabe der Erfindung, ein Konzept zur großflächigen Mucosa-Koagulation oder Mucosa-Ablation anzugeben.

Diese Aufgabe wird mit dem Ablationssystem nach Anspruch 1 gelöst:
Das erfindungsgemäße Ablationssystem umfasst eine Plasmaablationssonde mit wenigstens zwei gleichartigen Funken-Plasma-Elektroden, die zueinander in einem festen Abstand benachbart angeordnet sind. Sie werden von einer einzigen elektrischen Quelle mit HF-Spannung versorgt. Dazu dienen zwei elektrische parallel zueinander zu den Funken-Plasma-Elektroden geführte Leitungen, sowie eine Schalteranordnung, die zwischen der Quelle und den Leitungen angeordnet ist, um die Funken-Plasma-Elektroden abwechselnd mit HF-Spannung zu versorgen. Außerdem umfasst das Ablationssystem eine Gasquelle und zumindest eine Fluidleitung, die von der Gasquelle zu den Funken-Plasma-Elektroden führt.

Bei dem erfindungsgemäßen Ablationssystem gehen von der Ablationssonde wenigstens zwei Plasmastrahlen aus, die direkt nebeneinander auf das zu behandelnde Gewebe auftreffen. Die Plasmastrahlen werden abwechselnd energiebeaufschlagt (erregt), d.h. die beiden Funken-Plasmaelektroden zünden alternierend. Die beiden Plasmastrahlen können sich zu einem Strahl mit abgeflachtem Ovalquerschnitt, Streifenquerschnitt oder dergleichen vereinigen. Wenn der Plasmastrahl quer zur Längserstreckung seines Querschnitts über das Gewebe führt, wird ein breiter Gewebestreifen koaguliert. Durch das intermittierende Zünden der Funken an den beiden Funken-Plasmaelektroden kann einerseits mit hoher Spannung und hohem Strom und deswegen hoher Augenblicksleistung gearbeitet werden und andererseits die mittlere Leistung so begrenzt werden, dass die Ablationstiefe auf ein gewünschtes Maß beschränkt wird und Schädigungen tieferliegenden Gewebes oder gar Perforation des behandelten Hohlorgans vermieden werden können.

Die genannten Effekte sind in besonderem Maße zu verzeichnen, wenn die Funken-Plasmaelektroden in einem sich in distaler Richtung öffnenden spitzen Winkel voneinander weg weisend angeordnet sind. Damit wird die Behandlungsbreite maximiert, wobei die Außenmaße der Plasma-Ablationssonde so gering wie möglich, und dabei auf jeden Fall geringer sind, als bei parallel angeordneten Funken-Plasmaelektroden, die eine vergleichbare Behandlungsbreite erzeugen würden.

Besonders bevorzugt wird außerdem eine Ausführungsform, bei der die Ablationssonde zwei benachbarte Gasaustrittsdüsen aufweist, wobei in jeder Gasaustrittsdüse jeweils eine der Funken-Plasmaelektroden angeordnet ist. Die Gasaustrittsdüsen sind vorzugsweise in einem sich in distaler Richtung öffnenden spitzen Winkel voneinander weg weisend angeordnet, so dass die erzeugten Plasmastrahlen leicht divergieren. Es ist dabei auch möglich, die beiden Funken-Plasmaelektroden in einer einzigen Gasaustrittsdüse anzuordnen, die beispielsweise unrund, zum Beispiel länglich als Schlitzdüse ausgebildet sein kann, um einen fächerförmigen Plasmastrahl zu erzeugen. Unabhängig davon, kann die Ablationssonde mit einer einzigen Fluidleitung zur Gasversorgung oder auch mit zwei gesonderten Fluidleitungen versehen sein, die zu ihrer Gasversorgung dienen. Damit sind verschieden geometrisch bauliche Konzepte verwirklichbar.

Die beiden Funken-Plasmaelektroden werden von zwei elektrischen Leitungen mit Spannung versorgt, die vorzugsweise elektrisch miteinander koppelnd angeordnet sind. Zum Beispiel kann die Kopplung eine kapazitive Kopplung sein, die dadurch gegeben ist, dass die beiden elektrischen Leitungen in geringem Abstand zueinander parallel geführt sind, so dass zwischen beiden Leitungen (d.h. der jeweils spannungsführenden Leitung und der potentialfrei geschalteten Leitung) eine Kapazität von wenigstens einigen Picofarad vorteilhaft 45 Picofarad (pF) zu messen ist. Durch die elektrische Kopplung der beiden Leitungen miteinander wird beim Ablationsbetrieb bei schnellem Umschalten der Schalteranordnung eine vollständige Rekombination des Plasmas vor der jeweils abgeschalteten Elektrode und somit eine Spannungsspitze an dieser Elektrode beim Wiederzünden vermieden. Dies vergleichmäßigt und stabilisiert den Betrieb der Ablationssonde und verbessert das Koagulationsergebnis.

Die Schalteranordnung kann zum Beispiel mit einer Frequenz zwischen 1 und 20 Hz, vorzugsweise mit einer Frequenz von 5 Hz fortwährend umschalten, so dass die beiden Funken-Plasma-elektroden abwechselnd mit der HF-Spannungsquelle verbunden werden. Dabei wird eine Leitung mit der HF-Spannungsquelle verbunden und die jeweils andere Leitung spannungslos (potentialfrei) geschaltet. Die Schalteranordnung ist vorzugsweise an dem Generator angeordnet. Durch die (kapazitive) Kopplung beider Leitungen fließt in der jeweiligen Abschaltphase ein geringer kapazitiver Strom über die abgeschaltete Funken-Plasmaelektrode, der eine vollständige Rekombination des Plasmas während dieser kurzen Betriebsphase verhindert.

Beim Umschalten der Schalteranordnung können kurzzeitig beide Funken-Plasmaelektroden mit der HF-Quelle verbunden sein. Die Vorionisation des Plasmas an der bislang inaktiven Elektrode erleichtert während dieser kurzen Phase die Wiederaufnahme des Betriebs der zuvor kurzzeitig inaktiven Funken-Plasmaelektrode trotz der infolge des Betriebs der anderen Elektrode relativ geringen Spannung. Erfolgt das Umschalten hingegen geringfügig lückend, d.h. in einem kurzen Moment, in dem keine der Elektroden mit der HF-Elektrode verbunden ist, zündet die zuvor inaktive Elektrode leicht und ohne dass die HF-Spannungsquelle dabei eine zu große Spannungsspitze ausbildet. Damit werden Funkendurchschläge, die zu einer ungewünschten Gewebeschädigung führen könnten, vermieden.

Die Ablationssonde wird typischerweise endoskopisch eingesetzt. Dazu wird sie vorzugsweise an einem Endoskop außerhalb des Lumens desselben befestigt und mittels eines Adapters an dessen distalen Ende gehalten.

Das Endoskop und die Ablationssonde können in verschiedenen Lumina eines Kunststofffolienschlauchs angeordnet sein. Das Endoskop bleibt für andere Funktionen verfügbar, d.h. für andere Instrumente, für das Zuführen oder Abführen von Fluiden, für die optische Inspektion und/oder für die Beleuchtung des Operationsgebiets.

Einzelheiten vorteilhafter Ausführungsformen der Erfindung sind Gegenstand der nachfolgenden Beschreibung, der Zeichnung sowie von Ansprüchen. Es zeigen:
Figur 1 ein erfindungsgemäßes Ablationssystem mit einer Ablationssonde an einem Endoskop im Einsatz, in schematischer Darstellung,
Figur 2 das distale Ende des Endoskops nach Figur 1 mit darauf aufgesetzter Ablationssonde, in ausschnittsweiser Seitenansicht,
Figur 3 das Endoskop mit der Ablationssonde nach Figur 2, in perspektivischer Ansicht,
Figur 4 das Endoskop und die Ablationssonde nach Figur 2 und 3, in Stirnansicht,
Figur 5 die Ablationssonde, in ausschnittsweiser Schnittdarstellung,
Figur 6 eine abgewandelte Ausführungsform einer Ablationssonde, in schematischer längsgeschnittener Darstellung während des Betriebs bei Einwirkung auf die Mucosa,
Figur 7 das Ablationssystem als elektrisches Blockschaltbild,
Figur 8 das Ablationssystem, in schematisierter elektrischer Darstellung,
Figur 9 ein Zeitdiagramm zur schematisierten Veranschaulichung des Betriebs der Schalteranordnung des Ablationssystems nach Figur 7 und 8.

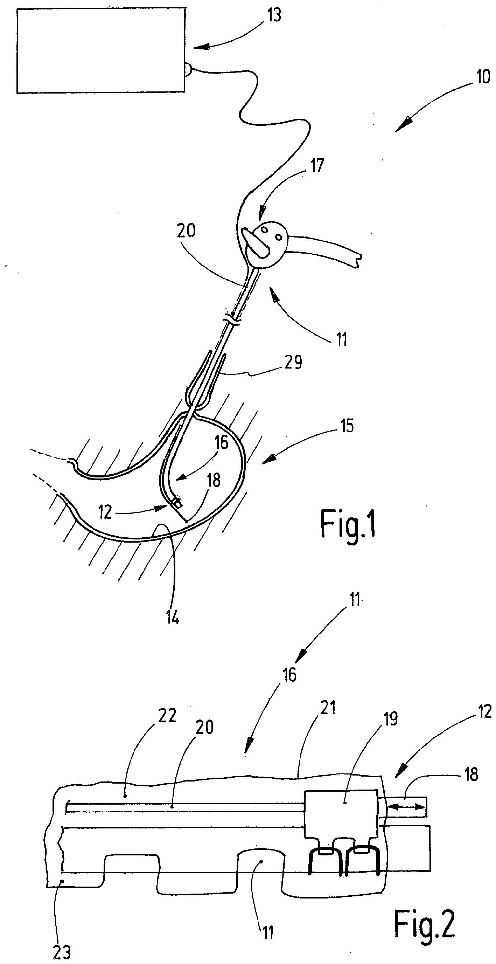

In Figur 1 ist ein Ablationssystem 10 veranschaulicht, zu dem ein Endoskop 11 mit einer Ablationssonde 12 und ein Gerät 13 zur Bereitstellung von Medien und Betriebsenergie für die Ablationssonde 12 und gegebenenfalls weiterer Instrumente gehören. Die Ablationssonde 12 dient zur Behandlung, insbesondere Koagulation und/oder Ablation, der Innenhaut eines Hohlorgans, insbesondere der Mucosa 14 eines Magens 15. Dazu wird das distale Ende 16 des Endoskops 11 mittels Bedienelementen 17 so geführt und gekrümmt, dass die Ablationssonde 12, insbesondere ein an deren distalen Ende vorgesehener Kopf 18, im gewünschten Abstand vorteilhaft senkrecht zu der Mucosa 14 steht und entlang derselben bewegt wird.

Das distale Ende 16 des Endoskops 11 und der Ablationssonde 12 sind in Figur 2 gesondert dargestellt. Das Endoskop 11 ist als ein- oder mehrlumiges mittels der Bedienelemente 17 gezielt krümmbares Rohr ausgebildet. An dem distalen Ende 16 desselben ist ein Adapter 19 gehalten, der zu der Ablationssonde 12 gehört. Weiter gehören zu der Ablationssonde 12 der Kopf 18, der wie in Figur 2 durch einen Doppelpfeil kenntlich gemacht, in Längsrichtung des Endes 16 des Endoskops 11 beweglich ist, sowie ein zug- und drucksteifer Schlauch 20, beispielsweise eine Fluidleitung 20 der mit dem Kopf 18 fest verbunden ist und sich parallel zu dem Endoskop 11 erstreckt. Der Schlauch 20 führt, wie Figur 1 erkennen lässt, zu dem Gerät 13. Das Endoskop 11 und der Schlauch 20 können von einer Schlauchhülle 21 aufgenommen sein, wobei sich die Ablationssonde 12 durch ein erstes Lumen 22, der Schlauchhülle 21 und das Endoskop 11 durch ein zweites Lumen 23 der Schlauchhülle 21 erstrecken. Damit kann die Gesamtanordnung, bestehend aus Endoskop 11, Ablationssonde 12 und Schlauchhülle 21, wie ein gewöhnliches Endoskop durch eine Speiseröhre 24 in den Magen 15 eines Patienten eingeführt und dort in ihre Bewegung gesteuert werden.

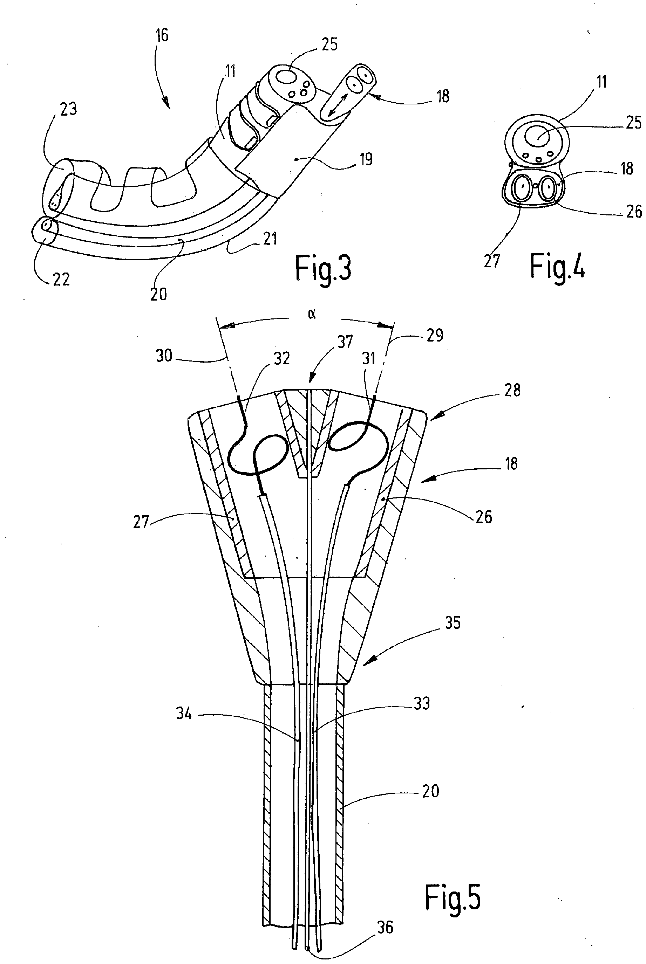

Figur 3 veranschaulicht das insoweit beschriebene Ende 16 des Endoskops 11 nochmals perspektivisch. Wie ersichtlich, kann das Endoskop 11 ein oder mehrere Lumen 25 sowie Bildübertragungsmittel, Beleuchtungseinrichtungen und dergleichen, enthalten. Wie Figur 3 zeigt, kann der Adapter 19 durch geeignete Klemmmittel oder sonstige Befestigungsorgane an dem Ende des Endoskops 11 befestigt sein. Figur 4 lässt erkennen, dass der Kopf 18 einen unrunden Querschnitt aufweisen kann, wobei an diesen zwei Gasaustrittsdüsen 26, 27 vorgesehen sein können. Diese können beispielsweise als keramische Einlagen ausgebildet sein. Zumindest an dem distalen Ende 28 des Kopfs 18 weisen die Gasaustrittsdüsen 26, 27 Öffnungsachsen 29, 30 auf, die miteinander vorzugsweise einen spitzen Winkel α einschließen. Dieser liegt vorzugsweise zwischen 10° und 60° und beträgt weiter vorzugsweise 10° bis 30°, insbesondere im Wesentlichen 25°.

Konzentrisch zu den Austrittsachsen 29, 30 sind Funken-Plasmaelektroden 31, 32 vorgesehen, die beispielsweise durch nadel- oder stiftförmige Wolframkörper oder durch sonstige elektrisch leitende, thermisch stabile Elemente gebildet sind. Die zentrischen in den Gasaustrittsdüsen angeordneten Funken-Plasma-Elektroden 31, 32 sind vorzugsweise ebenfalls in einem spitzen Winkel von vorzugsweise 10° bis 60°, vorzugsweise 10° bis 30°, insbesondere 25° angeordnet. Der Abstand der Funken-Plasmaelektroden 31, 32 voneinander beträgt vorzugsweise 5 mm bis 10 mm, vorzugsweise 7,5 mm. Die Funken-Plasma-Elektroden 31, 32 können vollständig innerhalb der Gasaustrittsdüsen 26, 27 angeordnet oder teilweise aus diesen herausragend angeordnet sein, wie es in Figur 5 dargestellt ist.

Die Funken-Plasmaelektroden 31, 32 sind jeweils mit einer elektrischen Leitung 33, 34 verbunden, deren Leiter mit einer elektrischen Isolierung, d.h. einem Dielektrikum versehen ist. Die Leitungen 33,34 erstrecken sich beide nebeneinander herlaufend durch das Lumen des Schlauchs 20, der an das proximale Ende 35 des Kopfes 18 zur Gasversorgung der Gasaustrittsdüsen 26, 27 angeschlossen ist. Die Leitungen können miteinander verdrillt sein, als Stegleitung ausgebildet sein oder in dem Schlauch 20 lose nebeneinander liegen.

Außerdem kann in dem Schlauch 20 eine Fluidleitung 36 vorgesehen sein, die dazu dient, einer an dem distalen Ende des Kopfs 28 vorgesehenen Flüssigkeitsejektionsdüse 37 Flüssigkeit, zum Beispiel Wasser (NaCl-Lösung), zuzuführen. Die Fluidleitung 36 kann alternativ außerhalb des Schlauchs 20 an dessen Außenseite entlang geführt sein. Die Flüssigkeitsejektionsdüse 37 kann dazu genutzt werden, die Mucosa 14 mit einer Flüssigkeit, insbesondere NaCl-Lösung zu unterspritzen, um diese von darunter liegendem Gewebe, insbesondere der Submucosa 51, abzuheben. Dazu können ein oder mehrere blasenartige Flüssigkeitsdepots unter der Mucosa 14 angelegt werden. Dies kann z.B. vor der Applikation von HF-Strom erfolgen. Vor thermischer Ablation beispielhaft der Mukosa 42 kann durch die Austrittsöffnung 37 Flüssigkeit so in die Magenwand eingebracht werden, dass sich ein Flüssigkeitskissen vorteilhaft unter der gewünschten Ablationsstelle bildet.

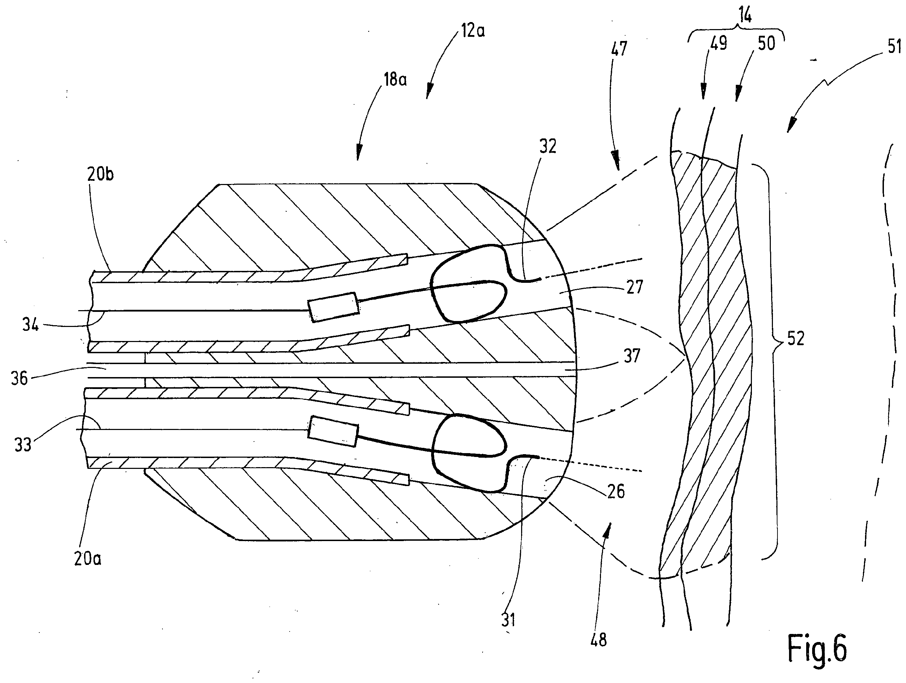

In Figur 6 ist eine abgewandelte Ausführungsform der Ablationssonde als Ablationssonde 12a veranschaulicht. Diese weist einen Kopf 18a auf, dessen Gasaustrittsdüsen 26, 27 durch gesonderte zug- und druckfeste Schläuche 20a, 20b mit Gas versorgt sind. Die Funkenplasmaelektroden 31, 32 können wiederum aus den Gasaustrittsdüsen 26, 27 herausschauend oder wie dargestellt in diesen versenkt angeordnet sein. Die elektrischen mit den Funken-Plasmaelektroden 31, 32 verbundenen Leitungen 33, 34 können isoliert oder wie dargestellt als nackte Leiter durch die Schläuche 20a, 20b geführt sein, die parallel nebeneinander herlaufend an dem Endoskop 11 entlang führen und sich durch das Lumen 22 erstrecken (entsprechend Figur 3). Im Übrigen gelten die zur Beschreibung der Ablationssonde 12 im Zusammenhang mit den Figuren 1 bis 5 gegebenen Erläuterungen entsprechend.

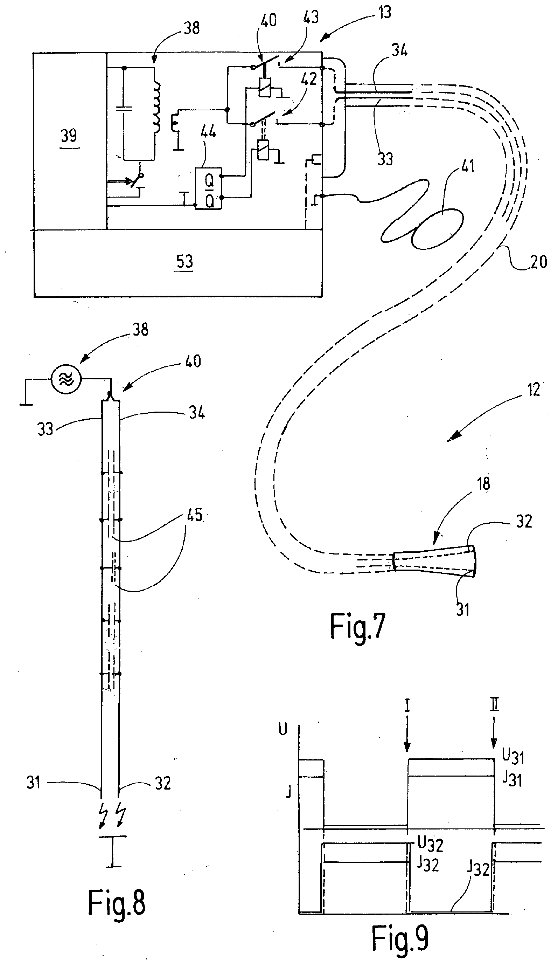

Zur Versorgung der Ablationssonde 12 dient das in Figur 7 näher veranschaulichte Gerät 13. Das Gerät 13 enthält eine elektrische Quelle 38, beispielweise einen HF-Generator 38, der typischerweise mit einer Frequenz zwischen 100 kHz und einem MHz, vorzugsweise mit einer Frequenz von zum Beispiel 300 kHz bis 400 kHz, insbesondere 350 kHz schwingt. Seine Energieversorgung erhält er aus einem Netz- und Steuerteil 39.

Aus dem HF-Generator 38 ausgekoppelte hochfrequente Wechselspannung wird über eine Schalteranordnung 40 abwechselnd an die Leitungen 33, 34 und somit abwechselnd an die Funken-Plasma-Elektroden 31, 32 gegeben. Die Schalteranordnung 40 kann Teil des Geräts 13 oder in einem gesonderten Zwischenmodul untergebracht sein, das an das Gerät 13 angeschlossen ist und an das die Leitungen 33, 34 angeschlossen sind.

Die abwechselnd an den Leitungen 33, 34 anliegende HF-Spannung hat einen Spitzenwert von vorzugsweise mehreren Tausend Volt (Zum Beispiel 5000 V), so dass sie in der Lage ist, an den Funken-Plasmaelektroden 31, 32 Funken zu zünden, die zu dem gegenüber liegenden biologischen Gewebe, zum Beispiel der Mucosa 14 (Figur 6), überspringen. Zur Stromrückführung ist eine Neutralelektrode 41 vorgesehen, die mit dem Gerät 13 verbunden und großflächig an dem Patienten zu befestigen ist.

Die Schalteranordnung 40 umfasst einen ersten Schalter 42, der die Leitung 33 mit dem HF-Generator 38 verbinden kann, und einen zweiten Schalter 43, der die Leitung 34 mit dem HF-Generator 38 verbinden kann. Die Schalter beziehungsweise Schließkontakte 42, 43 sind zum Beispiel Teil eines Relais oder zweier getrennter Relais, die gegensinnig schalten. Zur Ansteuerung kommt ein Taktsignal T von dem Netz- und Steuerteil. Das Signal T wird von einem Verstärker- und Inverterblock 44 zur gegensinnigen Ansteuerung der Schalter 42, 43 bewegenden Antriebe genutzt.

Der erste Schalter 42 und der zweite Schalter 43 arbeiten alternierend, so dass der Schalter 42 offen ist, wenn der Schalter 43 geschlossen ist und umgekehrt. Zu den Umschaltzeitpunkten I, II (Figur 9) können die beiden Schalter 42, 43 kurzzeitig beide geschlossen sein oder bei lückendem Betrieb auch kurzzeitig beide offen sein. Die Zeit der überlappenden Schließung (beide geschlossen) oder Schaltlücke (keiner geschlossen) ist kurz im Vergleich zur gesamten Schließzeit jedes Impulses. Das Umschalten erfolgt mit 1 Hz bis 20 Hz, vorzugsweise 5 Hz. Die elektrischen Leitungen 33, 34 erstrecken sich in enger Nachbarschaft durch das Lumen des Schlauchs 20 (oder alternativ durch zwei Schläuche 20a, 20b gemäß Figur 6) von dem Gerät 13 bis zu dem Kopf 18.

Die elektrischen Verhältnisse sind in Figur 8 veranschaulicht. Die parallel geführten Leitungen 33, 34 sind typischerweise deutlich länger als 1 m (zum Beispiel 2 m bis 5 m) lang. Somit koppeln die beiden Leitungen 33, 34 mit eine Kapazität größer 1 pF miteinander wie in Figur 8 durch gestrichelte Kondensatoren veranschaulicht ist. Diese sind keine diskreten Bauelemente, sondern sollen die zwischen beiden Leitungen 33, 34 vorhandene Koppelkapazität symbolisieren. Diese liegt typischerweise im Bereich zwischen 1 und 200 pF und hat Einfluss auf den Betrieb der Ablationssonde 12. Die vorhandene Koppelkapazität 45 schließt nicht aus, dass weitere Koppelelemente zum Beispiel ohmsche Widerstände, Kondensatoren und/ oder dergleichen zwischen beiden Leitungen 33, 34 angeordnet sind.

Zur Veranschaulichung des Betriebs der Ablationssonde 12 wird nachfolgend auf die Figuren 6, 8 und 9 Bezug genommen. Dabei wird zunächst davon ausgegangen, dass dem Kopf 18 über den Schlauch 20 Gas, z.B. Argon, aus einer Gas-Quelle 53 zugeführt wird, die Teil des Geräts 13 oder gesondert bereitgestellt sein kann.

Außerdem stellt die mit einer Frequenz von zum Beispiel 5 Hz alternierend umschaltende Schalteranordnung 40 in der betrachteten Phase eine Verbindung zwischen dem HF-Generator 38 und der Leitung 34 her. Die HF-Spannung liegt somit an der Funken-Plasmaelektrode 32 an. Es entsteht vor der Funken-Plasmaelektrode 32 ein Plasmastrahl 47, der sich vor der Gasaustrittsdüse 27 etwas erweitert und auf die Mucosa 14 trifft. In Figur 9 ist dies durch die Spannung U32 und den Strom I32 symbolisiert. Beide sind von Null verschieden und ergeben eine erhebliche Leistung von deutlich über 100 W.

Zum Umschaltzeitpunkt I schaltet die Schalteranordnung 40 um. Es wird nun die Spannung des Generators 38 an die Leitung 33 und somit an die Funken-Plasmaelektrode 31 gelegt. Es bildet sich nunmehr vor der Gasaustrittsdüse 26 ein Plasmastrahl 48, der wiederum auf biologisches Gewebe beispielsweise auf die Mucosa 14 trifft. Es liegt an der Funken-Plasmaelektrode 31 die Spannung U31 an und es fließt der Strom I31. Durch die kapazitive Kopplung fällt der Strom I32 jedoch nicht auf exakt Null. Vielmehr fließt ein geringer Teil des in der Leitung 33 fließenden Koagulationsstroms über die Koppelkapazität 45 auf die Leitung 32, so dass in der Umgebung der Funken-Plasmaelektrode 32 eine Restionisierung aufrechterhalten werden kann. Bei erneuter Umschaltung der Schalteranordnung 40 erleichtert dies zum Zeitpunkt II das erneute Zünden des Funkens an der Funken-Plasmaelektrode 32. Somit wird verhindert, dass der Generator 38 erst eine erhöhte Spannung aufbauen muss, um die Durchbruchsspannung für den Funkendurchschlag aufzubringen oder zumindest wird das Erfordernis einer solchen Spannungserhöhung vermindert. Es kann damit erreicht werden, dass die Energieabgabe in den beiden Plasmastrahlen 47, 48 vergleichmäßigt wird, womit die Behandlung durch den Behandler besser kontrollierbar wird.

Wie auch aus Figur 6 hervorgeht, können verschiedene Schichten 49, 50 der Mucosa 14 zuverlässig koaguliert und darunter liegende Schichten 51, hier im Besonderen die Muscualris geschont werden. Außerdem ist die Energieverteilung in jedem Plasmastrahl 47, 48 relativ gleichmäßig, so dass beide Strahlen insgesamt eine breite Koagulationsspur 52 von 10 bis 18 mm Breite hinterlassen, wenn der Kopf 18, 18a quer zu einer die Funken-Plasmaelektroden 31, 32 verbindenden Linie (in Figur 6 senkrecht zur Zeichenebene) entlang der Mucosa 14 geführt wird.

Das erfindungsgemäße Ablationssystem 10 arbeitet mit einer Ablationssonde 12, 12a, die zwei alternierend arbeitende Funken-Plasmaelektroden 31, 32 aufweist. Es wird von diesen ein Plasmastrahl 47, 48 mit nicht kreisförmigem Querschnitt erzeugt, der quer zur großen Längsachse seines vorzugsweise ovalen Querschnitts über abzutragendes Gewebe, insbesondere über die Mucosa 14 zu führen ist. Damit lassen sich großflächige Gewebebereiche prozesssicher und gut kontrollierbar bearbeiten.

**Bezugszeichen:**

| | |
|---|---|
| 10 | Ablationssystem |
| 11 | Endoskop |
| 12, 12a | Ablationssonde |
| 13 | Gerät |
| 14 | Biologisches Gewebe, Mucosa |
| 15 | Magen |
| 16 | distales Ende des Endoskops 11 |
| 17 | Bedienelemente des Endoskops 11 |
| 18, 18a | Kopf |
| 19 | Adapter |
| 20, 20a, 20b | zug- und drucksteifer Schlauch, Fluidleitung |
| 21 | Schlauchhülle |
| 22 | erstes Lumen der Schlauchhülle 21 |
| 23 | zweites Lumen der Schlauchhülle 21 |
| 24 | Speiseröhre |
| 25 | Lumen des Endoskops 11 |
| 26, 27 | Gasaustrittsdüsen |
| 28 | distales Ende des Kopfs 18 |
| 29, 30 | Öffnungs achsen |
| α | Winkel zwischen den Öffnungsachsen 30, 31 |
| 31, 32 | Funken-Plasmaelektroden |
| 33, 34 | Leitungen |
| 35 | proximales Ende des Kopfs 18 |
| 36 | Fluidleitung |
| 37 | Flüssigkeits-Ejektionsdüse |
| 38 | Elektrische Quelle, HF-Generator |
| 39 | Netz- und Steuerteil |
| 40 | Schalteranordnung |
| 41 | Neutralelektrode |
| 42 | erster Schalter, Schließkontakt |
| 43 | zweiter Schalter, Schließkontakt |
| 44 | Inverter- und Verstärkermodul |
| T | Takt |
| 45 | Koppelkapazität |
| 47, 48 | Plasmastrahl |
| 49, 50 | Schichten der Mucosa (lamina propria, muscularis mucosae) |
| 51 | Gewebe unterhalb der Mucosa (submucosa) |
| 52 | Koagulationsspur |
| 53 | Gas-Quelle |

## Patentansprüche

1. Ablationssystem (10), insbesondere zur großflächigen Mucosaablation,
mit einer Plasma-Ablationssonde (12, 12a), die wenigstens zwei gleichartige Funken-Plasmaelektroden (31, 32) aufweist, die einander benachbart angeordnet sind, mit einer einzigen elektrischen Quelle (38) für HF-Spannung und zwei elektrischen Leitungen (33, 34), die parallel zueinander zu den Funken-Plasmaelektroden (31, 32) führen, sowie einer Schalteranordnung (40), die zwischen der Quelle (38) und den zwei elektrischen Leitungen (33, 34) angeordnet ist, mit einer Gas-Quelle (53) und zumindest einer Fluidleitung (20), die von der Gas-Quelle (53) zu den gleichartigen Funken-Plasmaelektroden (31, 32) führt, und,
dass von der Ablationssonde wenigstens zwei Plasmastrahlen ausgehen, die direkt nebeneinander auf das zu behandelnde Gewebe auftreffen, **dadurch gekennzeichnet, dass** die Schalteranordnung angeordnet ist, um die Leitungen einander abwechselnd mit der Quelle zu verbinden, wobei die Plasmastrahlen abwechselnd energiebeaufschlagt werden und die beiden Funken-Plasmaelektroden alternierend zünden.

2. Ablationssystem nach Anspruch 1, **dadurch gekennzeichnet, dass** die Funken-Plasmaelektroden (31, 32) in einem sich in distaler Richtung öffnenden spitzen Winkel (α) voneinander weg weisend angeordnet sind.

3. Ablationssystem nach einem der vorstehenden Ansprüche, **dadurch gekennzeichnet, dass** die Ablationssonde zwei benachbarte Gasaustrittsdüsen (26, 27) aufweist, wobei in jeder Gasaustrittsdüse (26, 27) jeweils eine der Funken-Plasmaelektroden (31, 32) angeordnet ist.

4. Ablationssystem nach Anspruch 3, **dadurch gekennzeichnet, dass** die Gasaustrittsdüsen (26, 27) in einem sich in distaler Richtung öffnenden spitzen Winkel (α) voneinander weg weisend angeordnet sind.

5. Ablationssystem nach einem der vorstehenden Ansprüche, **dadurch gekennzeichnet, dass** zur Gasversorgung der Ablationssonde (12) eine einzige Fluidleitung (20) vorgesehen ist und dass die elektrischen Leitungen (33, 34) durch die Fluidleitung (20) geführt sind.

6. Ablationssystem nach einem der Ansprüche 1 bis 4, **dadurch gekennzeichnet, dass** zur Gasversorgung der Ablationssonde (12) zwei Fluidleitungen (20a, 20b) vorgesehen sind und dass in jeder Fluidleitung (20a, 20b) jeweils eine elektrische Leitung (33, 34) angeordnet ist.

7. Ablationssystem nach einem der vorstehenden Ansprüche, **dadurch gekennzeichnet, dass** die beiden elektrischen Leitungen (33, 34) elektrisch miteinander koppelnd angeordnet sind.

8. Ablationssystem nach einem der vorstehenden Ansprüche, **dadurch gekennzeichnet, dass** die beiden Leitungen (33, 34) miteinander kapazitiv koppelnd angeordnet sind.

9. Ablationssystem nach einem der vorstehenden Ansprüche, **dadurch gekennzeichnet, dass** die Schalteranordnung (40) zwei Schließkontakte (42, 43) aufweist, von denen zu jedem Zeitpunkt mindestens einer geschlossen ist.

10. Ablationssystem nach einem der vorstehenden Ansprüche, **dadurch gekennzeichnet, dass** die Frequenz, mit der die Schalteranordnung (40) umschaltet, zwischen 1 Hz und 20 Hz liegt und vorzugsweise 5 Hz beträgt.

11. Ablationssystem nach einem der vorstehenden Ansprüche, **dadurch gekennzeichnet, dass** die elektrische Quelle (38) eine Spannung zwischen 2000 V bis 5000 V mit einer Frequenz zwischen 100 kHz und 1 MHz und einer Leistung von 50 W bis 200 W bereitstellend ausgebildet ist.

12. Ablationssystem nach einem der vorstehenden Ansprüche, **dadurch gekennzeichnet, dass** zur Führung der Ablationssonde (12) ein Endoskop (11) vorgesehen ist.

13. Ablationssystem nach Anspruch 12, **dadurch gekennzeichnet, dass** das Endoskop (11) mindestens ein Lumen (25) aufweist und dass die Ablationssonde (12) an dem Endoskop (11) außerhalb des Lumens (25) desselben geführt ist.

14. Ablationssystem nach Anspruch 12 oder 13, **dadurch gekennzeichnet, dass** die Ablationssonde (12) mittels eines Adapters (19) an dem Endoskop (11) gehalten ist.

15. Ablationssystem nach einem der Ansprüche 12 bis 14, **dadurch gekennzeichnet, dass** das Endoskop (11) und die Ablationssonde (12) in verschiedenen Lumina (22, 23) einer Schlauchhülle (21) angeordnet sind.

## Claims

1. Ablation system (10), in particular for the large-scale mucosa ablation,
with a plasma ablation probe (12, 12a) having at least two identical spark plasma electrodes (31, 32) which are arranged next to one another,
with a single electrical source (38) for HF voltage and two electrical lines (33, 34) which lead parallel with one another to the spark plasma electrodes (31, 32), and a switch arrangement (40) arranged between the source (38) and the two electrical lines (33, 34),
with a gas source (53) and at least one fluid line (20) which leads from the gas source (53) to the identical spark plasma electrodes (31, 32), and
the ablation probe emits at least two plasma beams which meet the tissue to be treated directly next to one another,
**characterised in that** the switch arrangement is arranged so as to connect the lines alternately to the source, wherein the plasma beams are energised alternately and ignite the two spark plasma electrodes alternately.

2. Ablation system according to claim 1, **characterised in that** the spark plasma electrodes (31, 32) are arranged pointing away from one another at an acute angle (a) opening in the distal direction.

3. Ablation system according to any of the preceding claims, **characterised in that** the ablation probe has two adjacent gas outlet nozzles (26, 27), wherein a respective one of the spark plasma electrodes (31, 32) is arranged in each gas outlet nozzle (26, 27).

4. Ablation system according to claim 3, **characterised in that** the gas outlet nozzles (26, 27) are arranged pointing away from one another at an acute angle (a) opening in the distal direction.

5. Ablation system according to any of the preceding claims, **characterised in that** for gas supply to the ablation probe (12), a single fluid line (20) is provided and the electrical lines (33, 34) are conducted through the fluid line (20).

6. Ablation system according to any of claims 1 to 4, **characterised in that** for gas supply to the ablation probe (12), two fluid lines (20a, 20b) are provided and a respective electrical line (33, 34) is arranged in each fluid line (20a, 20b).

7. Ablation system according to any of the preceding claims, **characterised in that** the two electrical lines (33, 34) are arranged so as to be electrically coupled together.

8. Ablation system according to any of the preceding claims, **characterised in that** the two lines (33, 34) are arranged so as to be capacitatively coupled together.

9. Ablation system according to any of the preceding claims, **characterised in that** the switch arrangement (40) has two closing contacts (42, 43), at least one of which is closed at any time.

10. Ablation system according to any of the preceding claims, **characterised in that** the frequency with which the switch arrangement (40) switches is between 1 Hz and 20 Hz, and preferably amounts to 5 Hz.

11. Ablation system according to any of the preceding claims, **characterised in that** the electrical source (38) is configured to provide a voltage of between 2000 V and 5000 V, with a frequency between 100 kHz and 1 MHz, and a power of 50 W to 200 W.

12. Ablation system according to any of the preceding claims, **characterised in that** an endoscope (11) is provided in order to guide the ablation probe (12).

13. Ablation system according to claim 12, **characterised in that** the endoscope (11) has at least one lumen (25), and the ablation probe (12) is guided on the endoscope (11) outside the lumen (25).

14. Ablation system according to claim 12 or 13, **characterised in that** the ablation probe (12) is held on the endoscope (11) by means of an adapter (19).

15. Ablation system according to any of claims 12 to 14, **characterised in that** the endoscope (11) and the ablation probe (12) are arranged in different lumina (22, 23) of a hose sleeve (21).

## Revendications

1. Système d'ablation (10), destiné en particulier à l'ablation de muqueuse sur une grande surface,
comprenant une sonde d'ablation au plasma (12, 12a) qui présente au moins deux électrodes plasma à étincelles (31, 32) identiques, disposées de façon adjacente l'une à l'autre,
comprenant une source électrique (38) unique pour une tension HF et deux fils électriques (33, 34) qui mènent aux électrodes plasma à étincelles (31, 32), parallèlement l'un à l'autre, ainsi qu'un agencement de commutateur (40) qui est placé entre la source (38) et les deux fils électriques (33, 34),
comprenant une source de gaz (53) et au moins une conduite de fluide (20) qui mène de la source de gaz (53) aux électrodes plasma à étincelles (31, 32) identiques, et qu'au moins deux jets de plasma partent de la sonde d'ablation et tombent directement l'un à côté de l'autre sur le tissu à traiter,
**caractérisé en ce que** l'agencement de commutateur est disposé pour relier les fils en alternance à la source,
les jets de plasma étant chargés en énergie en alternance et amorçant en alternance les deux électrodes plasma à étincelles.

2. Système d'ablation selon la revendication 1, **caractérisé en ce que** les électrodes plasma à étincelles (31, 32) sont disposées de manière à s'éloigner l'une de l'autre, sous un angle aigu (a) qui s'ouvre dans la direction distale.

3. Système d'ablation selon l'une des revendications précédentes, **caractérisé en ce que** la sonde d'ablation présente deux buses de sortie de gaz (26, 27) voisines, respectivement l'une des électrodes plasma à étincelles (31, 32) étant disposée dans chaque buse de sortie de gaz (26, 27).

4. Système d'ablation selon la revendication 3, **caractérisé en ce que** les buses de sortie de gaz (26, 27) sont disposées de manière à s'éloigner l'une de l'autre, sous un angle aigu (a) qui s'ouvre dans la direction distale.

5. Système d'ablation selon l'une des revendications précédentes, **caractérisé en ce que** pour l'alimentation en gaz de la sonde d'ablation (12), il est prévu une seule conduite de fluide (20), et **en ce que** les fils électriques (33, 34) passent dans la conduite de fluide (20).

6. Système d'ablation selon l'une des revendications 1 à 4, **caractérisé en ce que** pour l'alimentation en gaz de la sonde d'ablation (12), il est prévu deux conduites de fluide (20a, 20b), et **en ce qu'**un fil électrique (33, 34) passe respectivement dans chaque conduite de fluide (20a, 20b).

7. Système d'ablation selon l'une des revendications précédentes, **caractérisé en ce que** les deux fils électriques (33, 34) sont disposés de manière à être couplés électriquement l'un à l'autre.

8. Système d'ablation selon l'une des revendications précédentes, **caractérisé en ce que** les deux fils (33, 34) sont disposés de manière à être couplés d'un point de vue capacitif l'un à l'autre.

9. Système d'ablation selon l'une des revendications précédentes, **caractérisé en ce que** l'agencement de commutateur (40) présente deux contacts de fermeture (42, 43) dont au moins un est fermé à tout instant.

10. Système d'ablation selon l'une des revendications précédentes, **caractérisé en ce que** la fréquence avec laquelle l'agencement de commutateur (40) commute est comprise entre 1 Hz et 20 Hz et est de préférence de 5 Hz.

11. Système d'ablation selon l'une des revendications précédentes, **caractérisé en ce que** la source électrique (38) est réalisée de manière à fournir une tension comprise entre 2 000 V et 5 000 V, avec une fréquence comprise entre 100 kHz et 1 MHz et une puissance allant de 50 W à 200 W.

12. Système d'ablation selon l'une des revendications précédentes, **caractérisé en ce qu'**un endoscope (11) est prévu pour le guidage de la sonde d'ablation (12).

13. Système d'ablation selon la revendication 12, **caractérisé en ce que** l'endoscope (11) présente au moins une lumière (25) et **en ce que** la sonde d'ablation (12) est guidée sur l'endoscope (11), à l'extérieur de la lumière (25) de celui-ci.

14. Système d'ablation selon la revendication 12 ou 13, **caractérisé en ce que** la sonde d'ablation (12) est maintenue sur l'endoscope (11) au moyen d'un adaptateur (19).

15. Système d'ablation selon l'une des revendications 12 à 14, **caractérisé en ce que** l'endoscope (11) et la sonde d'ablation (12) sont disposés dans des lumières (22, 23) différentes d'une gaine de tuyau (21).
